# EUROPÄISCHE PATENTSCHRIFT

(11) **EP 1 912 624 B1**
(45) Veröffentlichungstag und Bekanntmachung des Hinweises auf die Patenterteilung: **19.07.2017**
(21) Anmeldenummer: 06775843.3
(22) Anmeldetag: 04.08.2006
(51) Int. Cl.: A61Q 19/00, A61K 8/14, A61K 8/04, A61K 9/00, A61K 9/06, B01J 13/00, A61K 47/34, A61K 47/44, A61K 31/439, A61K 31/436

(54) **WASSERFREIES MEHR-PHASEN-GEL-SYSTEM**
ANHYDROUS MULTIPHASE GEL SYSTEM
SYSTEME GEL-MULTIPHASE ANHYDRE

(30) Priorität: 04.08.2005 DE 102005037844
(43) Veröffentlichungstag der Anmeldung: 23.04.2008
(60) Teilanmeldung: 11169258.8
(73) Patentinhaber: Intendis GmbH, 12529 Schönefeld (DE)
(72) Erfinder: FRANKE, Patrick, 14167 Berlin (DE)
(74) Vertreter: Gebauer, Olaf
(86) Internationale Anmeldenummer: PCT/DE2006/001410
(87) Internationale Veröffentlichungsnummer: WO 2007/014563

(56) Entgegenhaltungen:
- EP-A1- 0 344 040
- WO-A-01/64328
- WO-A-85/03640
- WO-A-92/09267
- WO-A1-00/61077
- DE-T2- 69 431 807
- FR-A- 2 848 879
- FR-A1- 2 681 248
- FR-A1- 2 787 730
- US-B1- 6 284 375
- M.WALLSTEN ET AL.: "Entrapment of lipid vesicles and membrane protein-lipid vesicles in gel bead pores" BIOCHIMICA ET BIOPHYSICA ACTA, Bd. 982, 1989, Seiten 47-52, XP009074774

## Beschreibung

Die vorliegende Erfindung betrifft eine topisch anwendbare Zusammensetzung in Form eines wasserfreien Mehr-Phasen-Gel-Systems.

Im Stand der Technik sind verschiedenste topisch anwendbare Zusammensetzungen bekannt. Zwei Vertreter dieser dermal anwendbaren Systeme sind Cremes und Salben.

Cremes sind Emulsionen, die eine dispergierte Phase und ein Dispersionsmittel umfassen. Man unterscheidet hierbei in wesentlichen zwischen Wasser-in-Öl- und Öl-in-Wasser-Emulsionen, je nachdem, welche Art von Emulgatoren eingesetzt werden. In jedem Fall handelt es sich jedoch um wasserhaltige Systeme.

Als besonders vorteilhaft empfinden viele Anwender von Cremes deren leichte Verteilbarkeit und die Tatsache, dass Cremes sich nicht klebrig und fettig auf der Haut anfühlen.

Ein Nachteil von Cremes ist jedoch, dass sie nur eine geringe Okklusion besitzen. Durch die Okklusion wird eine Hydratisierung der Hornschicht der Haut erreicht und die Hornschicht kann das Drei- bis Fünffache ihres Gewichts an Wasser aufnehmen und wird in diesem Zustand permeabler.

Weiterhin ist die Anwendung von Cremes als Vehikel aktiver Substanzen begrenzt, da das in Lotionen zwingend anwesende Wasser die Einbindung hydrolyseempfindlicher stark einschränkt oder sogar ausschließt.

Salben hingegen sind üblicherweise halbfeste Darreichungsformen aus Basis von Fettmaterial, die zur externen Applikation geeignet sind. Eine Salbenart sind wasserfreie Kohlenwasserstoff-Salben (Fettsalben), welche gerade oder verzweigte Kohlenwasserstoffe mit Kettenlängen von C₁₆ bis C₃₀ enthalten sowie auch cyclische Alkane beinhalten können. Eine typische Formulierung enthält flüssige Kohlenwasserstoffe (Mineralöle und flüssige Paraffine) gemischt mit Kohlenwasserstoffen mit längeren Alkylketten (mittlere Kettenlänge ca. 35 bis 50 Kohlenstoffatomen, üblicherweise n- und iso-Paraffine) mit hohen Schmelzpunkten, beispielsweise Vaseline, Hart-Paraffine und Wachse.

Vorteilhaft bei diesen wasserfreien Zusammensetzungen ist, dass sie eine hohe Okklusion auf der Haut aufweisen.

Nachteilig ist jedoch, dass Fettsalben zumeist sehr klebrig und fettig sind und deshalb bei vielen Anwendern ein unangenehmes Empfinden bei der Anwendung hervorrufen. Ferner weisen sie eine schlechte Verteilbarkeit auf der Haut auf.

Weiterhin ist die Anwendbarkeit dieser wasserfreien Salben als Freisetzungssystem für topisch zu verabreichende aktive Substanzen limitiert, weil viele aktive Substanzen eine relative schlechte Löslichkeit in einer Kohlenwasserstoff-Salbe besitzen. Dadurch ist die Inkorporation dieser Substanzen in diese topisch anwendbaren Systeme nur begrenzt und häufig nicht in einer wirksamen Konzentration möglich.

Bis zu einem bestimmten Maß kann das einer aktiven Substanz in der Kohlenwasserstoff-Salbe zur Verfügung stehende Lösemittelvolumen durch Verarbeitung der Salbengrundlagen mit Solventien erhöht werden, die mit Kohlenwasserstoffen mischbar sind, wie beispielsweise Isopropylmyristat. Hierdurch wird allerdings nur die Löslichkeit von aktiven Substanzen in der Fettsalbe gesteigert, die in mit Kohlenwasserstoffen mischbaren Lösemitteln löslich sind.

Um auch aktive Substanzen in die oben beschriebenen Kohlenwasserstoff-Salben zu inkorporieren, die in den mit Kohlenwasserstoffen mischbaren Lösemitteln nicht oder nur sehr begrenzt löslich sind, wurden in Kohlenwaserstoff-Salben Lösemittel eingebracht, welche nicht mit Kohlenwasserstoffen mischbar sind. Allerdings konnten bisher nur bis zu 5 % dieser Lösemittel in die klassischen Fettsalben eingebracht werden. Die Zubereitung wird sonst instabil (Synärese), d. h. es erfolgt ein "Ausschwitzen" der Lösemittel.

Ein weiterer wesentlicher Aspekt, der die Güte einer topisch anwendbaren Zusammensetzung bestimmt, ist dass sie ein hohes Maß an Penetration von enthaltenen kosmetischen, pflegenden und / oder therapeutischen Bestandteilen in die Haut bewirkt.

Es ist bekannt, dass sich die Penetration durch den Einsatz von Penetrationsförderern (Enhancern) steigern lässt. So wirken Substanzen, welche die polaren Komponenten der Hautlipide solvatisieren, zum Beispiel Wasser, Dimethylsulfoxid (DMSO) und Ethanol, durch die resultierende Erhöhung des Volumens der Lipidschichten der Haut sowohl für hydrophile als auch lipophile aktive Substanzen penetrationsfördernd. Stoffe, die mit den unpolaren Komponenten der Hautlipide interagieren, können die Mikrofluidität der Membranen beeinflussen und damit ebenfalls die Penetration verbessern. Dazu zählen Isopropylmyristat, Isopropylpalmitat und Ölsäure. Höhere Alkohole wie Propylenglykol, Glycerol und Sorbitol können sich direkt in die Wasserschichten zwischen den Lipiddoppelmembranen einlagern und verbessern hier die Löslichkeit vieler aktiver Substanzen, was häufig ebenfalls die Penetration erhöht.

Dabei sind viele unterschiedliche Einflüsse des Vehikels auf das Stratum corneum möglich, die sich auf die Permeation einer aktiven Substanz durch die Haut auswirken können. In den meisten Fällen kommt es zu einer Penetrationsbeschleunigung, die überwiegend durch die folgenden Mechanismen erklärt wird: Am häufigsten tritt eine Interaktion des Vehikels mit den interzellulären Lipiden im Stratum corneum auf, die entweder zu einer Lipidfluidisierung oder sogar zu einem Herauslösen einiger Lipidfraktionen aus dem Stratum corneum führen kann. Interaktionen mit den Lipiden sind je nach Eigenschaft des Vehikels mit den polaren Kopfgruppen und/oder mit den lipophilen Fettsäureresten der Lipide möglich. Außerdem führt das Eindringen größerer Mengen Vehikel in die Barriere zu einem Cosolventieneffekt. Voraussetzung ist, dass Vehikelbestandteile in der Lage sind in die Haut einzudringen und dabei die aktive Substanz mit einschleppen.

Neben dem Eindringen von Vehikel ist auch die verstärkte Hydratisierung des Stratum corneum unter okklusiven Bedingungen zu berücksichtigen und wie oben beschrieben, zeigen die im Stand der Technik bekannten Kohlenwasserstoff-Salben eine starke Okklusion. Die Penetrationsbeschleunigung kommt in allen Fällen durch eine Erhöhung des Diffusionskoeffizienten der wirksamen Substanz im Stratum corneum und/oder durch eine Erhöhung der Sättigungskonzentration der aktiven Substanz in der Barriere zu Stande.

Hat eine aktive Substanz nur eine geringe Neigung, aus der topisch anwendbaren Zusammensetzung in die Haut überzugehen, muss die Zusammensetzung so geändert werden, dass dennoch eine optimale dermale Verfügbarkeit erreicht wird.

Aufgabe der vorliegenden Erfindung ist es deshalb, eine topisch anwendbare Zusammensetzung bereitzustellen, welche die vorteilhaften Okklussionseigenschaften einer Fettsalbe besitzt, einer Creme oder auch Lotion ähnliche Anwendungseigenschaften zeigt, und zusätzlich als Vehikel mit hohem Penetrationsvermögen für eine Vielzahl, auch hydrolyseempfindlicher, aktiver Substanzen fungieren kann. Die Erfinder bezeichnen das erfindungsgemäße System mit dem Akronym "EDRS" oder "EDR-System", als Abkürzung für "Entrapped Drug Reservoir System".

Erfindungsgemäß wird die Aufgabe gelöst durch die Bereitstellung einer Zusammensetzung in Form eines wasserfreien Mehr-Phasen-Gel-Systems bestehend aus äußerer Lipidmatrix und einer mittels Polymer gelierten inneren Phase, dadurch gekennzeichnet, dass die Lipidphase hautverträgliche Lipid enthält, weiterhin dadurch gekennzeichnet, dass man die quellbaren Polymere oder Polymergemische mittels OH-Gruppen-haltigen Quellmittel quillt, weiterhin dadurch gkennzeichnet, dass das Quellmittel weiterhin Kohlensäurediester oder Mischungen aus Kohlensäurediestern umfasst erhältlich durch
a) Aufschmelzen der Lipidphase unter Bildung einer flüssigen Lipidphase,
b) Mischen und Homogenisieren von quellbaren Polymeren oder Polymergemischen unter Bildung einer zu dispergierenden Polymerphase, die OH-Gruppen-haltige Quellmittel sowie weiterhin Kohlensäurediester oder Mischungen aus Kohlendiestern umfasst
c) Vereinigen der Polymerphase mit der flüssigen Lipidphase und Homogenisierung der Phasen und
d) Kaltrühren des Phasengemisches bis zur Ausbildung einer festen gelartigen Misch-Struktur des Gesamtsystems (EDRS).

Erfindungsgemäß bevorzugt ist ein System, wobei die Lipidphase hautverträgliche Lipide enthält.

Besonders bevorzugt ist, dass die Lipide ausgewählt sind aus Petrolatum, Paraffin, mikrokristallinem Wachs, Squalen, Cetylstearyloctanoat, Ethyloleat, Glyceryltricaprylat/caprat, Myristylmyristat, Propylenglycoldicaprat, Cetylestern, Isopropylmyrisat, Isopropylpalmitat, Mono-, Di- und Triglyceriden, ethoxylierten Glyceriden, Polyethylenglycolestern, Sorbitanestern, Hartfett, Hartfett (Novata^{™}), Dibutyladipat, Ethyllinoleat, Glycol Isoceteth-3 Acetat (Crodamole) wie E-thylhexylcocoat Isocetylstearat, Oleyloleat (Cetiol^{™}) Cetylpalmitate, Cetylpalmitat (Cutina CP^{™}) Cetylalkohol, Oleylalkohol, Stearylakohol, Dicaprylylether, Ölsäure, Wachsen, wie Jojobawachs und Bienenwachs, Cholesterinen, Polyethylenglykolen, Lanolin, Lanolinalkoholen, Silikonölen und deren Mischungen Bevorzugt ist ferner ein erfindungsgemäßes System, wobei es sich bei den Polymeren um Cellulosederivate, Acrylatpolymere und deren Derivate oder deren Mischung handelt.

Besonders bevorzugt ist auch, dass es sich bei den Cellulosederivaten um Hydroxypropylcellulose, Carboxymethylcellulose, Methylcellulose, Hydroxypropylmethylcellulose, Hydroxyethylcellulose und deren Derivate oder deren Mischung handelt.

Insbesondere bevorzugt ist es auch, dass es sich bei den Acrylatpolymeren um quervernetzte Acrylatpolymere handelt.

Bevorzugt ist dabei, dass das Quellmittel ein- bis dreiwertige aliphatische Alkohole mit einer Kettenlänge von bis zu 5 Kohlenstoffatomen oder deren Mischungen sind.

Ganz besonders bevorzugt ist auch, dass die einwertigen aliphatischen Alkohole ausgewählt sind aus Ethanol, n-Propanol und Isopropanol oder deren Mischung.

Außerdem ist erfindungsgemäß bevorzugt, dass die Polyole ausgewählt sind aus Glycerin, Propylenglykol und 1,2-Pentandiol oder deren Mischung. Das Quellmittel umfasst Kohlensäuredieseter oder Mischungen aus Kohlensäurediestern.

Besonders bevorzugt ist hierbei, dass die Kohlensäurediester ausgewählt sind aus der Gruppe Ethylencarbonat, Propylencarbonat und weiteren Homologen von Ethylencarbonat und deren Mischungen.

Weiterhin ist bevorzugt, dass das Quellmittel weiterhin Diethylenglykolmonoethylether, polyoxylierte Caprylcaprinsäureglyceride, Dimethylisosorbid und/oder weitere pharmazeutisch verträgliche Lösemittel oder deren Mischungen umfasst.

Ganz besonders bevorzugt ist ein erfindungsgemäßes System, wobei die Lipidphase und/oder die Polymerphase aktive Bestandteile enthält.

Bevorzugt ist auch, dass die Lipidphase und die Polymerphase verschiedene aktive Bestandteile enthalten. Besonders bevorzugt ist es, dass die aktiven Bestandteile ausgewählt sind aus hautpflegenden Substanzen, hautfärbenden Substanzen, UV-Protektoren, pharmazeutisch wirksamen Substanzen oder deren Mischungen.

Bevorzugte aktive Bestandteile sind beispielsweise ausgewählt aus Polidocanol, synthetischen Gerbstoffen, Antiseptika, wie Chlorhexidin und Triclosan, Antibiotika, wie Fusidinsäure, Erythromycin, Tetracykline, Clindamycin, Peptidantibiotika, Antimykotika, wie Imidazolderivate, Terbenafin, Ciclopirox, Salizylsäure, Zink-Pyrithion, topische Kortikosteroide, wie z.B. Methylprednisolonaceponat, Clobetasol, Mometasonfuorat, topische Makrolide, wie Tacrolimus und Pimecrolimus, Oligonukleotide zur Gentherapie, wie si-RNA und Ribozyme, Antihistaminika, Immunsuppressiva, wie Cyclosporin, Azathioprin und Mycophenolatmofetil, Anthralinen, wie Cignolin und Dithranol, Vitamin D3-Analoga, wie Calcipotriol, topische Retinoide, Harnstoff, Milchsäure, Fumarsäureester, Azelainsäure, Hydrochinon, Benzoylperoxid, nicht-steroidale Antiphlogistika, Sexualhormone, wie Östrogene und Androgene, Zytostatika, UV-Protektoren, wie Stilbenderivaten, Makeup (Camouflage), pflanzlichen Extrakten, wie Grünteeextrakt, Centella asiatica Extrakt, Weidenrindenextrakt, Birkenextrakt, Grünteeextrakt, Teebaumöl, Olivenblattextrakt, A-loe vera Extrakt, Ringelblumenextrakt, Passionsblütenextrakt, Hamamelisextrakt, Kamillenextrakt, Teebaumöl, Bärentraubenblätterextrakt und Süßholzwurelextrakt, beispielsweise als 18ß-Glycyrhetinsäure (Zn Kombination), Fruchtsäuren, wie α-Hydroxysäuren, β-Hydroxysäuren und Polyhydroxysäuren (PHA), Äpfelsäure, Malonsäure, Citronensäure, oder deren Mischungen. Die Aufzählung der verwendbaren Wirkstoffe ist nicht abschließend. Natürlich können auch andere Wirkstoffe in das erfindungsgemäß System eingebracht werden.

Besonders bevorzugt ist es, dass der aktive Bestandteil gemeinsam mit einem Solubilisierungsmittel eingebracht wird.

Ein bevorzugtes erfindungsgemäßes System umfasst weiterhin ein oder mehrere für eine topisch anwendbare Zusammensetzungen nützliche Additive umfasst.

Ein weiterer Gegenstand der vorliegenden Erfindung ist die Verwendung eines erfindungsgemäßen Systems zur Herstellung einer pharmazeutischen Zusammensetzung zur Anwendung auf der Haut, den Schleimhüten und/oder auf Wundflächen.

Bevorzugt ist dabei die Verwendung zur Herstellung eines human- oder veterinärmedizinischen Erzeugnisses, also zur Anwendung bei Menschen und bei Tieren.

Ein weiterer Gegenstand der vorliegenden Erfindung ist ein Verfahren zur Herstellung einer Zusammensetzung in Form eines wasserfreien Mehr-Phasen-Gel-Systems bestehend aus äußerer Lipidmatrix und einer mittels Polymer gelierten inneren Phase, dadurch gekennzeichnet, dass die Lipidphase hautverträgliche Lipid enthält, weiterhin dadurch gekennzeichnet, dass man die quellbaren Polymere oder Polymergemische mittels OH-Gruppen-haltigen Quellmittel quillt, weiterhin dadurch gkennzeichnet, dass das Quellmittel weiterhin Kohlensäurediester oder Mischungen aus Kohlensäurediestern umfasst wobei man
a) die Lipidphase unter Bildung einer flüssigen Lipidphase aufschmilzt,
b) quellbare Polymere oder Polymergemische unter Bildung einer zu dispergierenden Polymerphase mischt und homogenisiert, wobei die Polymerphase die OH-Gruppen-haltige Quellmittel sowie weiterhin Kohlensäurediester oder Mischungen aus Kohlendiestern umfasst
c) die Polymerphase mit der flüssigen Lipidphase vereinigt und die Phasen homogenisiert und
d) das Phasengemisch bis zur Ausbildung einer festen gelartigen Misch-Struktur des Systems kaltrührt.

Bevorzugt ist ein Verfahren, wobei man der Polymerphase, der Lipidphase oder beiden Phasen einen Wirkstoff zusetzt.

Überraschenderweise wurde gefunden, dass die erfindungsgemäßen Zusammensetzungen ausgesprochen vorteilhafte Eigenschaften besitzen. Sie weisen eine mit einer Fettsalbe vergleichbaren Okklusion auf und besitzen zusätzlich die positiven Anwendungseigenschaften einer Creme, wie angenehmes Hautgefühl beim Anwender und leichte Verteilbarkeit auf der Haut.

Weiterhin hat sich gezeigt, dass die erfindungsgemäßen Zusammensetzungen als topisch anzuwendende Vehikel für unterschiedlichste Substanzklassen besonders geeignet sind.

Die erfindungsgemäßen Zusammensetzungen gestatten die Inkorporation unterschiedlichster Stoffklassen in wirksamen Mengen. Der aktive Stoff kann entweder in die äußere Lipidmatrix oder in die gelierte dispergierte Phase, die ein Lösungsreservoir bereitstellt, aufgenommen werden. Und weiterhin können unterschiedliche aktive Stoffe in einer erfindungsgemäßen Zusammensetzung enthalten sein, indem sie gemeinsam in eine Phase eingebracht werden, oder auf die beiden Phasen des Systems verteilt werden.

Und auch die Penetration der in der erfindungsgemäßen Zusammensetzung enthaltenen kosmetischen, pflegenden und/oder therapeutischen Substanzen in die Haut hat sich als ausgesprochen vorteilhaft erwiesen.

Diese überraschenden Eigenschaften der erfindungsgemäßen Zusammensetzungen ergeben sich offensichtlich dadurch, dass ein stabiles Mehr-Phasen-Gel-System gebildet wird.

Der Vorteil einer solchen Kompartimentierung in Form einer kohärenten Lipid-Phase und einer darin eingeschlossenen gelierten, dispergierten Phase, die ein Reservoir ausbildet, hat sich beispielsweise dadurch gezeigt, dass sich die erfindungsgemäße Zusammensetzung u. a. hervorragend als Lösungsvehikel für hydrophobe aktive Substanzen eignet, die in Kohlenwasserstoffen in wirksamen Konzentrationen wenig löslich oder praktisch unlöslich sind oder aufgrund ihrer Hydrolyseempfindlichkeit nicht mit Wasser in Berührung kommen dürfen (z.B. TIMS (z. B. Tacrolimus, Pimecrolimus, Ascrolimus), Korticoide und Hormone, Antibiotika). Dies wurde mittels einer induzierten Kontaktdermatitis an Mäusen nachgewiesen.

Hierbei ist es insbesondere vorteilhaft, dass die erfindungsgemäße Zusammensetzung die stabile Inkorporation von nicht-wässerigen Lösungsmitteln wie z.B. Propylencarbonat, Propylenglycol, Glycerin im System bis zu Konzentrationen von 30 % und die Aufnahme von Ethanol und anderen kurzkettigen Alkoholen bis zu 25 % gestattet.

Damit steht aktiven Substanzen ein wesentlich größeres Lösungsmittelreservoir zur Verfügung, verglichen mit im Stand der Technik bekannten topisch anwendbaren Systemen mit vergleichbaren Anwendungseigenschaften.

Ein wesentlicher Vorteil der erfindungsgemäßen Mehr-Phasen-Gel-Zusammensetzung liegt hierbei also in der effektiven Lösungsvermittlung von schwerlöslichen aktiven Substanzen, die in klassischen Kohlenwasserstoff-Salben in wirksamen Konzentrationen meist nicht möglich ist.

Und es hat sich auch gezeigt, dass die besonderen Eigenschaften des erfindungsgemäßen Systems die dermale Zuführung von aktiven Substanzen, die nur eine geringe Tendenz zeigen, aus einer Zusammensetzung in die Haut einzudringen, beim Einsatz von Lösungsvermittlern oder Lösungsmittelgemischen im Vergleich zu im Stand der Technik bekannten Systemen verbessern.

Allerdings ist nach dem bekannten Stand der Technik der Einsatz von Lösungsvermittlern oder Lösemittelgemischen, welche die Sättigungslöslichkeit einer aktiven Substanz in der Formulierung erhöhen, bei den im Stand der Technik bekannten Cremes und Kohlenwasserstoff-Salben dadurch begrenzt, dass davon nur begrenzte Mengen Penetrationsverstärker in die Systeme inkorporiert werden können, ohne dass die Stabilität des Systems dramatisch reduziert wird.

Diese Eigenschaft wurde insbesondere dann beobachtet, wenn sich der Lösungsvermittler und die aktive Substanz in die innere Phase des Systems eingebracht wurden, die als diskretes Kompartiment in der erfindungsgemäßen Zusammensetzung vorliegt.

Bei dem erfindungsgemäßen System wird hingegen ein eigenes Lösemittel-Kompartiment erzeugt. Hierin liegen Penetrationsverstärker und Wirkstoff in hohen Konzentrationen und enger räumlicher Nähe vor, was zu einer besseren Löslichkeit des Wirkstoffs an sich im System und folglich auch zu einer besseren Penetration durch die Haut führt.

Es wird davon ausgegangen, dass dadurch das Konzentrationsgefälle der aktiven Substanz zunimmt, wodurch gleichzeitig die Penetrationsgeschwindigkeit steigt. Somit erreicht man im erfindungsgemäßen System durch den Zusatz der Penetrationsverbesserer eine erhöhte Wirkstoffkonzentration in tieferen Hautschichten, da diese Substanzen ihrerseits in die Haut diffundieren und die Barrierefunktion der Hornhaut reduzieren.

Die Steigerung der Penetration ist möglich durch die Wahl der Art und der Menge des Lösemittels, in Abhängigkeit der eingesetzten Wirkstoffe und der Wahl der Konzentration und des Ortes der Beladung des Systems.

Es sind also in den erfindungsgemäßen Systemen beide wesentliche Aspekte der Penetrationsverbesserung von aktiven Substanzen in die Haut gegeben, Okklusionseffekt und Penetrationsverstärkung durch hautgängige verträgliche Lösemittel, welche die inkorporierten aktiven Substanzen in die Haut befördern.

Im Sinne dieser Erfindung bezeichnet ein Mehr-Phasen-Gelsystem ein System, welches aus zwei und mehr Phasen gebildet ist.

Im Zusammenhang der vorliegenden Erfindung bezieht sich der Begriff "aktive Substanz" auf eine Substanz, die einen gewünschten kosmetischen, pflegenden und / oder therapeutischen Effekt auf der Haut direkt oder den Organismus des Anwenders insgesamt ausübt.

Erfindungsgemäß bezieht sich der Begriff "wirksame Konzentration" auf die Konzentration, die erforderlich ist, um den gewünschten kosmetischen, pflegenden und / oder therapeutischen Effekt zu bewirken.

Die Verwendung des Begriffs "kosmetischer Effekt" im vergleich zum "pflegenden Effekt" soll hervorheben, dass die erfindungsgemäßen Zusammensetzungen nicht nur physiologisch den Zustand der Haut verbessern können, sondern auch im Bereich der dekorativen Kosmetik einsetzbar sind. Beispielhaft hierfür ist neben der dekorativen Bedeckung der Haut als Makeup auch die Abdeckung von Hautstellen wie Muttermalen, Narben und Hautstörungen, wie sie häufig als Folge von Erkrankungen, wie beispielsweise AIDS auftreten, als so genanntes "Camouflage".

Wasserfrei im Sinne der vorliegenden Erfindung bedeutet, dass bis zu 1 % Wasser in der Zusammensetzung enthalten sein können. Wasserfreie Lösemittel, können erfindungsgemäß bis zu 5 % Wasser enthalten. So hat sich beispielsweise gezeigt, dass Ethanol für die erfindungsgemäße Verwendung bis zu 4,5 % Wasser (Azeotrop) enthalten kann.

Die mittels OH-Gruppen quellbaren Polymere können ausgewählt werden aus Acrylatpolymeren oder deren Mischungen. Beispielhaft seien hier einige Produkte der Firma Noveon Inc. genannt:
Carbopol 934 NF, Carbopol 934P NF, Carbopol 940 NF, Carbopol 971P NF, Carbopol 71G NF, Carbopol 974P NF, Carbopol 980 NF, Carbopol 981 NF, Carbopol 1342 NF, Carbopol 5984 EP, Pemulen TR-1 NF, Pemulen TR-2 NF, Noveon AA-1 USP, Noveon CA-1 USP und Noveon CA-2 USP.

Im Zusammenhang der vorliegenden Erfindung bezieht sich der Begriff kurzkettige Alkohole auf ein- bis dreiwertige aliphatische Alkohole mit bis zu fünf Kohlenstoffatomen.

Sämtliche Verfahrensschritte zur Herstellung der erfindungsgemäßen Zusammensetzungen können durch Techniken erfolgen, die einem Durchschnittsfachmann bekannt sind. So kann das Mischen und Homogenisieren der Bestandteile in Schritt b) zur Erzeugung der dispergierten Polymerphase durch gängige Rührsysteme und Homogenisatoren erfolgen.

Die Einstellung der Viskosität der erzeugten Polymerphase kann durch beispielsweise Erwärmen erfolgen.

Neben den bereits aufgeführten Bestandteilen, kann die erfindungsgemäße Zusammensetzung weiterhin ein oder mehrere, für eine topisch anwendbare Zusammensetzungen nützliche Additive umfassen. Diese Additive können beispielsweise ausgewählt sein aus Farbstoffen, Geruchsstoffen, Konservierungsmitteln und absorptionsfördernden Mitteln.

Nachfolgend werden Ausführungsbeispiele der Erfindung dargelegt und Untersuchungsergebnisse vorgestellt, welche die Eigenschaften und Vorteile der erfindungsgemäße Zusammensetzungen, auch im Vergleich mit herkömmlichen topisch anwendbaren Systemen zeigen.

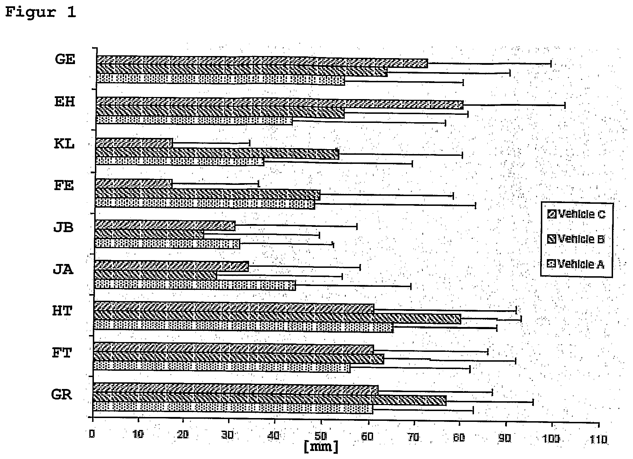

An Hand der Figuren 1 bis 5 werden die experimentellen Ergebnisse erläutert.

Es zeigt:
Figur 1
   Ergebnisse einer Anwendungsstudie auf menschlicher Haut (Messwert: Hautgefühl allgemein) gemäß Beispiel 7
Figur 2
   Ergebnisse einer Anwendungsstudie auf menschlicher Haut (Messwert: Wirkung bei Läsionen) gemäß Beispiel 7
Figur 3
   Ergebnisse einer Anwendungsstudie auf menschlicher Haut (Messwert: Gesamtwirkung) gemäß Beispiel 7
Figur 4
   Ergebnisse einer induzierten Kontaktdermatitis bei Mäusen (Messwert: Ohrgewicht) gemäß Beispiel 8
Figur 5
   Ergebnisse einer induzierten Kontaktdermatitis bei Mäusen (Messwert: Peroxidase) gemäß Beispiel 8

Die nachfolgenden Beispiele erläutern die Erfindung ohne diese jedoch einzuschränken. Es wird ausdrücklich beansprucht, dass vorteilhafte Wirkungen in den nachfolgenden Beispielen nur zur Veranschaulichung dargestellt werden können, ohne eine abschließende Aufzählung darstellen zu wollen.

### Beispiele 1 bis 5

Die folgende Tabelle 1 zeigt beispielhafte Rezepturen (ad 100 g) für das erfindungsgemäße wasserfreie Mehr-Phasen-Gel-System auf Basis von halbfesten Lipidformulierungen mit gelierten Mischungen aus Propylencarbonat und Propylenglycol oder Ethanol. Tabelle 1 zeigt erfindungsgemäße Zusammensetzungen an Hand der Beispiele 1 bis 5. Die erste Spalte gibt die bei der Herstellung verwendeten Bestandteile wieder. Die zweite Spalte gibt die Grenzen an, in welchen Mengen (in Gew.-%) die jeweiligen Bestandteile in der fertigen Formulierung vorliegen können. Die Spalten drei bis sieben stellen ausgewählte Rezepturen dar (Mengenangeben jeweils in Gew.-%). In den Beispielen 3 und 4 (Spalten fünf und sechs) enthalten die erfindungsgemäßen Zusammensetzungen als aktive Substanz Ascrolimus.

Tabelle 1 zeigt somit, dass die Mengen der einzelnen Bestandteile in weiten Grenzen variierbar sind, um die erfindungsgemäße Zusammensetzung mit den vorteilhaften Eigenschaften zu erhalten.

Es ist dem Fachmann daher ohne weiteres möglich, mit Hilfe weniger Versuche eine Rezeptur zu ermitteln, welche auch sehr schwer lösliche Wirkstoffe in der erfindungsgemäßen Zusammensetzung in Lösung bringt, um deren Applikation auf oder durch die Haut zu gewährleisten. Als Beispiel für einen Wirkstoff, der sich mit topischen Zubereitungen nach dem Stand der Technik fast nicht topisch applizieren lässt, ist in den Beispielen 3 und 4 dargestellt. Das topische Makrolid Ascrolimus ist hier in einer hoher Konzentration (bis zu 1,0 Gew.-%) in eine erfindungsgemäße Zusammensetzung eingebracht worden.

**Tabelle 1**

| **Bestandteil** | **Grenzen** | **Beispiel 1** | **Beispiel 2** | **Beispiel 3** | **Beispiel 4** | **Beispiel 5** |
|---|---|---|---|---|---|---|
| Ascrolimus (Wirkstoff) | 0 - 1,0 | - | - | 1,0 | 0,1 | - |
| Carbomer Copolymer (Pemulen TR-1/TR-2) | 0,1 - 0,6 | 0,45 | 0,3 | 0,15 | 0,15 | 0,40 |
| Carbomer (Carbopol 980) | 0 - 0,3 | 0,17 | - | - | - | 0,17 |
| Hydroxypropylcellulose (Klucel HF) | 0 - 0,4 | 0,12 | - | - | - | - |
| Hydroxyethylmethylcellulose (Tylopur MH 1000) | 0 - 0,4 | - | 0,15 | - | - | - |
| Propylencarbonat | 5,0 - 15,0 | 14,75 | 9,775 | 5,0 | 5,0 | - |
| Ethanol 96%ig | 0 - 20 | - | - | - | - | 19,0 |
| Propylenglykol | 5,0 - 15,0 | 14,75 | 9,775 | 5,0 | 5,0 | |
| Diisopropylamin | 0 - 0,1 | 0,075 | 0,024 | - | - | 0,05 |
| Weißes Vaselin | 40,0 - 80,0 | 47,685 | 57,976 | 65,85 | 66,75 | 59,95 |
| Paraffinöl, dickflüssig | 0 - 10,0 | 10,0 | 10,0 | 10,0 | 10,0 | 10,0 |
| Cyclomethicone | 0 - 10,0 | 3,0 | - | - | - | 3,0 |
| Bienenwachs (Cera Alba) | 0 - 5,0 | 2,0 | 5,0 | 5,0 | 5,0 | 2,0 |
| Hartparaffin | 0 - 7,0 | 7,0 | 7,0 | 5,0 | 5,0 | 7,0 |
| Mikrokristallines Wachs | 0 - 5,0 | - | - | 3,0 | 3,0 | - |

### Beispiel 6

### Allgemeine Herstelltechnologie für ein EDRS

Das nachstehend abgebildete Schema 1 zeigt die allgemeine Herstellungstechnologie für das erfindungsgemäße EDR-System. Die in das System einzubringenden Wirkstoffe können, je nach dem gewünschten Zweck, entweder in die Lipidphase oder in die Polymerphase, oder aber auch in beide Phasen eingebracht werden. Auch können die Temperaturen, bei denen die einzelnen Herstellungsschritte erfolgen, einen Einfluss ausüben. Die Lipide müssen fast immer aufgeschmolzen werden, während die Polymerphase unter besonderen Bedingungen der Zusammensetzung bereits hinreichend flüssig ist, so dass ein weiteres Verflüssigen, beispielsweise durch Erwärmen ausbleiben kann. Beim Vereinigen der Phasen kann dann die Vesikel-Größenverteilung in gewünschter Weise beeinflusst werden. Bei Anschließenden Abkühlen der Mischungen werden dann die Vesikel unter Gelbildung immobilisiert. Die einzelnen Herstellungsschritte sind dem Fachmann, einem Galeniker durchaus geläufig. Die entsprechenden Parameter zu ändern, um das optimale EDRS zu erhalten, ist somit dem Fachmann ohne weiteres möglich und geläufig. Das hier angegebene Herstellungsschema ist nicht als Beschränkung zu verstehen. Übliche Verfahrensvariationen werden als zum Umfang der Erfindung gehörend angesehen.

### Beispiel 7

### Anwendungsstudie auf der menschlichen Haut

Nachfolgend wird eine Untersuchung zur Verträglichkeit der erfindungsgemäßen Zusammensetzung im Vergleich mit zwei Formulierungen nach dem Stand der Technik beschrieben. Eingesetzt wurden die Zusammensetzungen zur topischen Behandlung atopischer Dermatitis über den Zeitraum von einer Woche an einer einzelnen Targetläsion. Durchgeführt wurde die Versuchsreihe als doppelblinde, randomisierte, Einzel-Center Zwei-Perioden Crossover-Untersuchung.

18 Probanden (weiblich und männlich, kaukasischer Typ im Alter von 19 bis 41 mit wenigstens zwei 4 cm² großen Läsionen) nahmen an der Studie bis zum Ende teil; eine statistische Verteilung fand nicht statt. Die Probanden erhielten innerhalb zweier aufeinander folgender Wochen zwei unterschiedliche Behandlungen an jeweils anderen Läsionen. Lediglich einzelne Anwendungen wurden von einzelnen Probanden ausgelassen.

### Anwendung fanden drei Zubereitungen:

Vehikel A: Fettsalbe (Stand der Technik)
Vehikel B: erfindungsgemäßes wasserfreies Mehr-Phasen-Gel-System (EDRS)
Vehikel C: Wasser-in-Öl-Zubereitung (Stand der Technik)

### Messwerte:

1. Die Probanden sollten das Hautgefühl einschließlich des Auftretens von Juckreiz auf einem Fragebogen beurteilen, wobei der Bereich der Beurteilung 0 bis 100 betrug.
2. Die Probanden sollten den Gesamteindruck der jeweiligen Behandlungen angeben.
3. Kommentare der Probanden jeder Art, spontan geäußert oder auf Nachfrage (alles was dem Probanden missfiel, jedes wahrgenommene Empfinden; Hautirritationen, Pickel/Ausschlag, Hauterwärmung, Kältegefühl, Stechen, Kribbeln, Brennen, Juckreiz, Hautspannung, Schmerzen, etc.) wurden gesammelt.
4. Bewertung der Gesamtschwere der Läsionen (Abschätzung der Zielläsionen durch den Arzt: Schwere des Erythems, Ödem/Papulation, Nässen/Verkrustung, Exkoration, Lichenifikation wurden abgeschätzt und auf einer 4-Punkte-Scala bewertet) wurde durch einen Arzt durchgeführt.
5. Fotodokumentation, Negativereignisreport, Blutdruck, Herzfrequenz wurden gemessen bzw. erstellt.

### Ergebnisse:

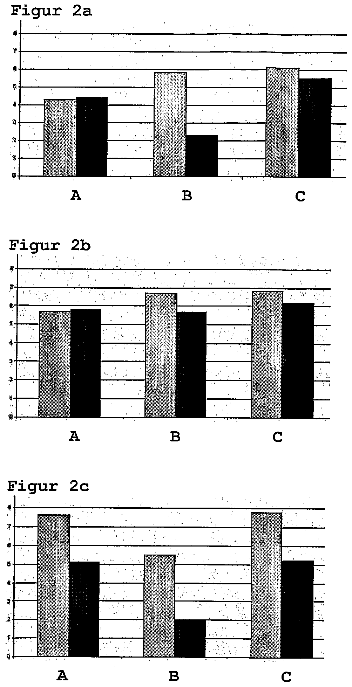

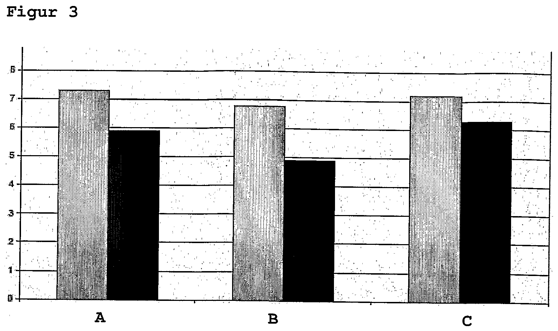

Ad 1. Die Ergebnisse sind in der Figur 1 dargestellt. Auf der x-Achse ist der Mittelwert (Balken) und die Standardabweichung (Strich) in mm aufgetragen.
   Die Probanden wurden nach folgenden Parametern befragt: Gesamteindruck (GE), Eindringen in die Haut (EH), Klebrigkeit (KL), Fettigkeit (FE), Juckreiz während der Behandlung (JB), Juckreiz beim Auftragen (JA), Hautrockenheit (HT), Feuchtigkeit (FT), Geruch (GR).
   Es wird deutlich, dass die erfindungsgemäße Zusammensetzung (Vehikel B) in den meisten Fällen mit der Wasser-in-Öl-Formulierung (Vehikel C), welche im Allgemeinen als besonders angenehm auf der Haut empfunden wird, vergleichbar ist. Besonders bemerkenswert ist, dass der Juckreiz, sowohl direkt beim Auftragen, als auch im Verlauf der Behandlung, als signifikant geringer, im Vergleich zu den anderen Vehikeln A und C, bewertet wurde.
Ad 2. Sieben Probanden bevorzugten insgesamt Vehikel C, fünf Probanden bevorzugten Vehikel A und gleichfalls fünf Probanden bevorzugten Vehikel B (Erfindungsgemäßes EDRS).
Ad 3. Von den Probanden wurde keine spontanen Kommentare abgegeben. Auf Nachfrage konzentrierten sich die Kommentare der Probanden auf drei Bereiche, nämlich Hautirritation, Pickel/Ausschlag und Juckreiz. In den Figur 2a bis 2c sind die Ergebnisse für die Messgrößen Hautirritation (Figur 2a), Pickel/Ausschlag (Figur 2b) und Juckreiz (Figur 2c) graphisch dargestellt. Der Bewertungsrahmen umfasst eine Skala (y-Achse) von 0 bis 12 und berücksichtigt die Intensität des Empfindens (0 - 3) und deren Dauer (1 - 4). Auf der x-Achse sind jeweils zwei Balken für die Vehikel A (A), Vehikel B (B) und Vehikel C (C) aufgetragen, wobei die jeweils linke Säule das Empfinden vor der Behandlung und die jeweils rechte Säule das Empfinden nach der Behandlung mit dem jeweiligen Vehikel angibt. Es zeigt sich, dass das erfindungsgemäße EDRS (Vehikel B) in allen Fällen die signifikant bessern Ergebnisse erzielte. Besonders deutlich ist hierbei der Behandlungseffekt bei Hautirritationen und beim Juckreiz.
Ad 4. Die Bewertung der Gesamtschwere der Läsionen ist in Figur 3 dargestellt. Auf der x-Achse sind jeweils zwei Balken für die Vehikel A (A), Vehikel B (B) und Vehikel C (C) aufgetragen. Die jeweils linken Säulen der Säulenpaare zeigen die Schwere vor der jeweiligen Behandlung. Die jeweils rechten Säulen geben die Schwere der Läsionen nach der Behandlung mit dem entsprechenden Vehikel wieder. Es zeigt sich deutlich, dass Vehikel B (erfindungsgemäßes EDRS) die signifikant beste Wirkung zeigte.
Ad 5. Während der Studie wurden drei Negativereignisse berichtet:
   Proband 1: Bronchitis, steht in keiner Beziehung zur Behandlung im Verlauf der Studie
   Proband 2: Urtikaria geringer Intensität, weit entfernt von den Läsionen, beim Probanden schon früher beobachtet Proband 8: Exacerbation der atopischen Dermatitis in der behandelten Läsion, vermutlich durch Vehikel C bedingt.

Zusammenfassend wird festgestellt, dass die klassische Wasser-in-Öl-Formulierung von den Probanden im Allgemeinen bevorzugt ist, Allerdings weist das erfindungsgemäße EDRS (Vehikel B) insbesondere bei den therapeutisch wichtigen Eigenschaften, wie Juckreiz, Hautirritationen und in der Gesamtwirkung, erheblich Vorteile gegenüber den Formulierungen des Standes der Technik auf.

### Beispiel 8

### DBFB induzierte Kontaktdermatitis an Mäusen

Um die Effektivität und Bioverfügbarkeit von Wirkstoffen in Abhängigkeit von der verwendeten topischen Arzneiform zu ermitteln kann man sich verschiedener Modelle bedienen. Ein geeignetes pharmakologisches Modell stellt im Rahmen anti-inflammatorischer Therapie an der Haut das 'Irritant contact dematitis (ICD)' Modell an Mäusen dar. Hierbei werden die inflammatorischen-Komponenten von Ekzemen imitiert. Dieser inflammatorische Aspekt spielt ebenso bei allergischer Kontaktdermatitis und atopischer Dermatitis unter Beteiligung Allergen-spezifischer Hauteigener T-Zellen eine Rolle. Daher kann ein T-Zellen abhängiges Modell allergischer Kontaktdermatitis (in Reaktion auf Dinitrofluorobenzol, DNFB) herangezogen werden.

Akute und chronische allergische Kontaktdermatitis (ACD) sind durch ein Typ 1 dominiertes Cytokin Profil charakterisiert. Deshalb wurde das Dinitrofluorobenzol (DNFB) induzierte ACD Modell benutzt, um in vivo die Aktivität des Wirkstoffes 'Ascrolimus' (topischer Immunmodulator) zu beurteilen. Nach Sensibilisierung mittels DNFB wurden durch nachfolgende Applikation mit demselben Kontaktallergen Ödeme erzeugt sowie eine kutane Infiltration von allergenen T-Tellen und Granulozyten provoziert.

Die Mäuse (NMRI) wurden mit 0.5% DNFB an Tag 0 und Tag 1 sensibilisiert. Am Tag 5 wurden die Mäuse mit 0.3% DNFB erneut behandelt. Die Testprodukte mit dem Wirkstoff Ascrolimus wurden in drei verschiedenen Konzentrationen (0.1, 0.3, 1.0%) in einer Standard-Fettsalbe sowie mit dem erfindungsgemäßen EDRS topisch co-appliziert. Nach 24 h wurden die Tiere geopfert, um das Ohrgewicht, Elastase und Peroxidase Aktivität von Ohr-Homogenisaten als Parameter für Ödeme und Granulozyten-Infiltration. Jedes Experiment wurde zweimal wiederholt.

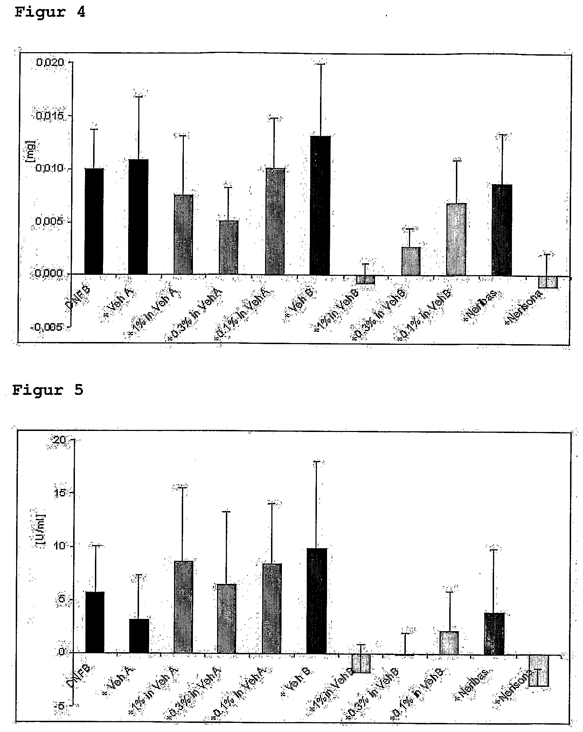

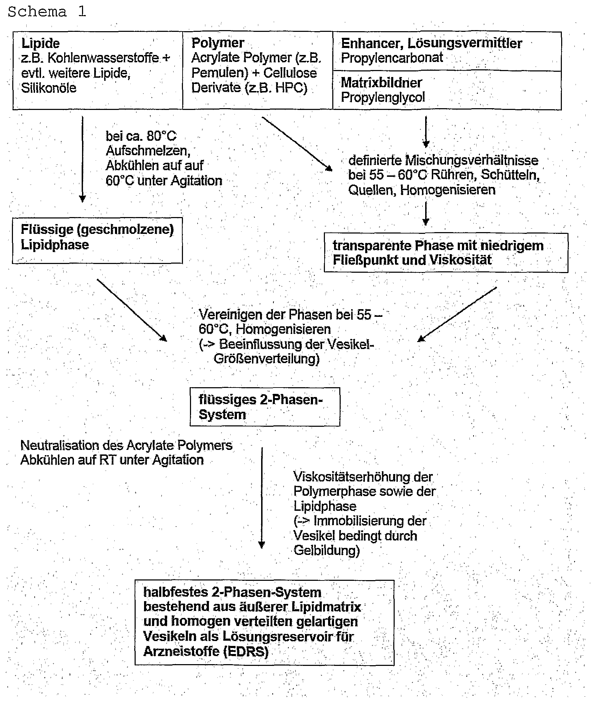

Die Ergebnisse der Ohrgewichtsmessung als Maß für die Ödembildung zeigen für die EDRS-Verum Salben, konzentrationsabhängig eine deutliche Reduktion der induzierten inflammatorischen Effekte gegenüber den Standard-Fettsalben. Hier zeigt sich bei gleicher 1%iger Konzentration von Ascrolimus keine signifikante Reduktion des Ohrgewichtes (y-Achse = delta Ohrgewicht in mg [mg]) im Vergleich zum Placebo (Figur 4).

Die Peroxidase-Aktivität als Maß für die Granulozyten-Infiltration (Figur 5) zeigte (y-Achse = delta Peroxidase in Einheiten pro ml [U/ml]) ebenso signifikant eine konzentrationsabhängige Hemmung, wenn Ascrolimus in im EDR-System appliziert wurde. Dem gegenüber konnte mit der Standard-Fettsalbe kein signifikanter Effekt gemessen werde. Lediglich der interne Standard (Glucocorticoid) zeigte auch eine Hemmung der Ödembildung sowie der Granulozyten-Infiltration.

Legende zu den Figuren 4 und 5:
Vehikel A = Standard-Fettsalbe (Placebo)
Vehikel B = EDRS (Placebo)
0.1, 0.3, 1.0% in Vehikel A oder B = Konzentrationen von Ascrolimus im Vehikel
Neribas = Grundlage (Placebo); Interner Standard Nerisona = Standard-Fettsalbe mit Diflucortolonpivalat (Glukocorticoid); Interner Standard

Es konnte beispielhaft gezeigt werden, dass die erfindungsgemäßen Systeme (EDRS) ausreichend stabil sind und eine hohe Tolerabilität aufweisen. In Abhängigkeit der physiko-chemischem Eigenschaften sowie der Potenz von Arzneistoffen, können das Lösemittelgemisch in Art und Menge im Kohlenwasserstoff-System innerhalb bestimmter Grenzen variiert werden.

## Patentansprüche

1. Zusammensetzung in Form eines Wasserfreien Mehr-Phasen-Gel-Systems bestehend aus äußerer Lipidmatrix und einer mittels Polymer gelierten inneren Phase, **dadurch gekennzeichnet, dass** die Lipidphase hautverträgliche Lipide enthält, weiterhin **dadurch gekennzeichnet, dass** man die quellbaren Polymere oder Polymergemische mittels OH-Gruppen-haltigen Quellmitteln quillt, weiterhin **dadurch gekennzeichnet, dass** das Quellmittel weiterhin Kohlensäuredieseter oder Mischungen aus Kohlensäurediestern umfasst erhältlich durch
a) Aufschmelzen der Lipidphase unter Bildung einer flüssigen Lipidphase,
b) Mischen und Homogenisieren von quellbaren Polymeren oder Polymergemischen unter Bildung einer zu dispergierenden Polymerphase, die OH-Gruppen-haltige Quellmittel sowie weiterhin Kohlensäurediester oder Mischungen aus Kohlensäurediestern umfasst
c) Vereinigen der Polymerphase mit der flüssigen Lipidphase und Homogenisierung der Phasen und
d) Kaltrühren des Phasengemisches bis zur Ausbildung einer festen gelartigen Misch-Struktur des Gesamtsystems.

2. Zusammensetzung gemäß Anspruch 1, **dadurch gekennzeichnet, dass** die Lipide ausgewählt sind aus Petrolatum, Paraffin, mikrokristallinem Wachs, Squalen, Cetylstearyloctanoat, Ethyloleat, Glyceryltricaprylat/caprat (wohl nicht 2 Verbindungen), Myristylmyristat, Propylenglycoldicaprat, Cetylestern, Isopropylmyrisat, Isopropylpalmitat, Mono-, Di- und Triglyceriden, ethoxylierten Glyceriden, Polyethylenglycolestern, Sorbitanestern, Hartfett (Novata™), Dibutyladipat, Ethyllinoleat, Propylen Glycol Isoceteth-3 Acetat(Crodamole), Ethylhexylcocoat, Isocetylstearat, Oleyloleat (Cetiol™), Cetylpalmitat, Cetylpalmitat (Cutina CP™), Cetylalkohol, Oleylalkohol, Stearylakohol, Dicaprylylether, Ölsäure, Wachsen, Jojobawachs, Bienenwachs, Cholesterinen, Polyethylenglykolen, Lanolin, Lanolinalkoholen, Silikonölen und deren Mischungen

3. Zusammensetzung gemäß einem der vorangehenden Ansprüche 1 bis 2, **dadurch gekennzeichnet, dass** es sich bei den Polymeren um Cellulosederivate, Acrylatpolymere oder deren Mischung handelt.

4. Zusammensetzung gemäß Anspruch 3, **dadurch gekennzeichnet, dass** es sich bei den Cellulosederivaten um Hydroxypropylcellulose, Carboxymethylcellulose, Methylcellulose, Hydroxypropylmethylcellulose, Hydroxyethylcellulose oder deren Mischung handelt.

5. Zusammensetzung gemäß Anspruch einem der Ansprüche 1 bis 3, **dadurch gekennzeichnet, dass** es sich bei den Acrylatpolymeren um quervernetzte Acrylatpolymere handelt.

6. Zusammensetzung gemäß einem oder mehreren der Ansprüche 1 bis 5, **dadurch gekennzeichnet, dass** das Quellmittel ein- bis dreiwertige aliphatische Alkohole mit einer Kettenlänge von bis zu 5 Kohlenstoffatomen oder deren Mischungen sind.

7. Zusammensetzung gemäß Anspruch 6, **dadurch gekennzeichnet, dass** die einwertigen aliphatischen Alkohole ausgewählt sind aus Ethanol, n-Propanol und Isopropanol oder deren Mischung.

8. Zusammensetzung gemäß Anspruch 6, **dadurch gekennzeichnet, dass** die Polyole ausgewählt sind aus Glycerin, Propylenglykol und 1,2-Pentandiol oder deren Mischung.

9. Zusammensetzung gemäß einem oder mehreren der Ansprüche 1 bis 8, **dadurch gekennzeichnet, dass** die Kohlensäurediester ausgewählt sind aus der Gruppe Ethylencarbonat, Propylencarbonat und weiteren Homologen von Ethylencarbonat und deren Mischungen.

10. Zusammensetzung gemäß einem der Ansprüche 6 bis 9, **dadurch gekennzeichnet, dass** das Quellmittel weiterhin Diethylenglykolmonoethylether, polyoxylierte Caprylcaprinsäureglyceride, Dimethylisosorbid und/oder weitere pharmazeutisch verträgliche Lösemittel oder deren Mischungen umfasst.

11. Zusammensetzung gemäß einem oder mehreren der Ansprüche 1 bis 10, **dadurch gekennzeichnet, dass** die Lipidphase und/oder die Polymerphase weiterhin aktive Bestandteile enthält.

12. Zusammensetzung gemäß Anspruch 11, **dadurch gekennzeichnet, dass** die Lipidphase und die Polymerphase verschiedene aktive Bestandteile enthalten.

13. Zusammensetzung gemäß Anspruch 11 oder Anspruch 12, **dadurch gekennzeichnet, dass** die aktiven Bestandteile ausgewählt sind aus hautpflegenden Substanzen, hautfärbenden Substanzen, UV-Protektoren, pharmazeutisch wirksamen Substanzen oder deren Mischungen.

14. Zusammensetzung gemäß Anspruch 11 oder 12, **dadurch gekennzeichnet, dass** die aktiven Bestandteile ausgewählt sind aus Polidocanol, synthetischen Gerbstoffen, Antiseptika, Chlorhexidin, Triclosan, Antibiotika, Fusidinsäure, Erythromycin, Tetracykline, Clindamycin, Peptidantibiotika, Antimykotika, Imidazolderivate, Terbenafin, Ciclopirox, Salizylsäure, Zink-Pyrithion, topische Kortikosteroide, Methylprednisolonaceponat, Clobetasol, Mometasonfuorat, topische Makrolide, Ascrolimus, Tacrolimus, Pimecrolimus, Oligonukleotide zur Gentherapie, si-RNA, Ribozyme, Antihistaminika, Immunsuppressiva, Cyclosporin, Azathioprin, Mycophenolatmofetil, Anthraline, Cignolin, Dithranol, Vitamin D3-Analoga, Calcipotriol, topische Retinoide, Harnstoff, Milchsäure, Fumarsäureester, Azelainsäure, Hydrochinon, Benzoylperoxid, nicht-steroidale Antiphlogistika, Sexualhormone, Östrogene, Androgene, Zytostatika, UV-Protektoren, Stilbenderivate, pflanzlichen Extraktewie Grünteeextrakt, Centella asiatica Extrakt, Weidenrindenextrakt, Birkenextrakt, Teebaumöl, Olivenblattextrakt, Aloe vera Extrakt, Ringelblumenextrakt, Passionsblütenextrakt, Hamamelisextrakt, Kamillenextrakt, Bärentraubenblätterextrakt und Süßholzwurelextrakt, 18β-Glycyrhetinsäure (Zn Kombination), Fruchtsäuren wie α-Hydroxysäuren, β-Hydroxysäuren, Polyhydroxysäuren oder deren Mischungen.

15. Zusammensetzung gemäß einem der Ansprüche 11 bis 14, **dadurch gekennzeichnet, dass** der aktive Bestandteil gemeinsam mit einem Solubilisierungsmittel eingebracht wird.

16. Zusammensetzung gemäß einem oder mehreren der Ansprüche 1 bis 15, **dadurch gekennzeichnet, dass** die Formulierung weiterhin ein oder mehrere für eine topisch anwendbare Zusammensetzungen nützliche Additive umfasst.

17. Verfahren zur Herstellung einer wasserfreien Mehr-Phasen-Gel-Zusammensetzung bestehend aus äußerer Lipidmatrix und einer mittels Polymer gelierten inneren Phase, **dadurch gekennzeichnet, dass** die Lipidphase hautverträgliche Lipide enthält, weiterhin **dadurch gekennzeichnet, dass** man die quellbaren Polymere oder Polymergemische mittels OH-Gruppen-haltigen Quellmitteln quillt, weiterhin **dadurch gekennzeichnet, dass** das Quellmittel weiterhin Kohlensäuredieseter oder Mischungen aus Kohlensäurediestern umfasst, wobei man
a) die Lipidphase unter Bildung einer flüssigen Lipidphase aufschmilzt,
b) quellbare Polymere oder Polymergemische unter Bildung einer zu dispergierenden Polymerphase mischt und homogenisiert, wobei die Polymerphase die OH-Gruppen-haltige Quellmittel sowie weiterhin Kohlensäurediester oder Mischungen aus Kohlendiestern umfasst
c) die Polymerphase mit der flüssigen Lipidphase vereinigt und die Phasen homogenisiert und
d) das Phasengemisch bis zur Ausbildung einer festen gelartigen Misch-Struktur des Systems kaltrührt.

18. Verfahren gemäß Anspruch 17, **dadurch gekennzeichnet, dass** man der Polymerphase, der Lipidphase oder beiden Phasen einen Wirkstoff zusetzt.

## Claims

1. Composition in the form of an anhydrous multiphase gel system consisting of an outer lipid matrix and an inner phase gelled by means of a polymer, **characterized in that** the lipid phase contains lipids that are compatible with the skin, further **characterized in that** the polymers or polymer blends that can be swollen are swollen by means of swelling agents containing OH groups, further **characterized in that** the swelling agent additionally comprises carbonic acid diesters or mixtures of carbonic acid diesters, which phase can be obtained by
a) melting the lipid phase with the formation of a liquid lipid phase,
b) mixing and homogenizing polymers or polymer blends capable of swelling with the formation of a polymer phase to be dispersed which comprises swelling agents containing OH groups and additionally carbonic acid diesters or mixtures of carbonic acid diesters,
c) combining the polymer phase with the liquid lipid phase and homogenizing the phases, and
d) cold stirring the phase mixture until a solid gel-like mixed structure of the entire system is formed.

2. Composition according to Claim 1, **characterized in that** the lipids are selected from petrolatum, paraffin, microcrystalline wax, squalene, cetylstearyl octanoate, ethyl oleate, glyceryl tricaprylate/caprate (but not 2 compounds), myristyl myristate, propylene glycol dicaprate, cetyl esters, isopropyl myristate, isopropyl palmitate, mono-, di- and triglycerides, ethoxylated glycerides, polyethylene glycol esters, sorbitan esters, solid lipids (Novata™), dibutyl adipate, ethyl linoleate, propylene glycol isoceteth-3 acetate (crodamols), ethylhexyl cocoate, isocetyl stearate, oleyl oleate (Cetiol™), cetyl palmitate, cetyl palmitate (Cutina CP™), cetyl alcohol, oleyl alcohol, stearyl alcohol, dicaprylyl ether, oleic acid, waxes, jojoba wax, beeswax, cholesterins, polyethylene glycols, lanolin, lanolin alcohols, silicone oils and their mixtures.

3. Composition according to either of the preceding Claims 1 and 2, **characterized in that** the polymers involve cellulose derivatives, acrylate polymers or their mixture.

4. Composition according to Claim 3, **characterized in that** the cellulose derivatives involve hydroxypropylcellulose, carboxymethylcellulose, methylcellulose, hydroxypropylmethylcellulose, hydroxyethylcellulose or their mixture.

5. Composition according to one of Claims 1 to 3, **characterized in that** the acrylate polymers involve crosslinked acrylate polymers.

6. Composition according to one or more of Claims 1 to 5, **characterized in that** the swelling agent is a monohydric to trihydric aliphatic alcohol with a chain length of up to 5 carbon atoms or mixtures thereof.

7. Composition according to Claim 6, **characterized in that** the monohydric aliphatic alcohols are selected from ethanol, n-propanol and isopropanol or mixtures thereof.

8. Composition according to Claim 6, **characterized in that** the polyols are selected from glycerin, propylene glycol and 1,2-pentanediol or mixtures therof.

9. Composition according to one or more of Claims 1 to 8, **characterized in that** the carbonic acid diesters are selected from the group: ethylene carbonate, propylene carbonate and other homologs of ethylene carbonate and mixtures thereof.

10. Composition according to one of Claims 6 to 9, **characterized in that** the swelling agent additionally comprises diethylene glycol monoethyl ether, polyoxylated capryl/capric acid glycerides, dimethyl isosorbide and/or additional pharmaceutically compatible solvents or mixtures thereof.

11. Composition according to one or more of Claims 1 to 10, **characterized in that** the lipid phase and/or the polymer phase additionally contain active components.

12. Composition according to Claim 11, **characterized in that** the lipid phase and the polymer phase contain different active components.

13. Composition according to Claim 11 or Claim 12, **characterized in that** the active components are selected from skin-care substances, skin-coloring substances, UV protectors, pharmaceutically active substances or mixtures thereof.

14. Composition according to Claim 11 or 12, **characterized in that** the active components are selected from polidocanol, synthetic tanning substances, antiseptics, chlorhexidine, triclosan, antibiotics, fusidic acid, erythromycin, tetracycline, clindamycin, peptide antibiotics, antimycotics, imidazole derivatives, terbenafine, ciclopirox, salicylic acid, zinc pyrithione, topical corticosteroids, methylprednisolone aceponate, clobetasol, mometasone fuorate, topical macrolides, ascrolimus, tacrolimus, pimecrolimus, oligonucleotides for gene therapy, si-RNA, ribozymes, antihistamines, immunosuppressants, cyclosporin, azathioprine, mycophenolate mofetil, anthralines, cignolin, dithranol, vitamin D3 analogs, calcipotriol, topical retinoids, urea, lactic acid, fumaric acid ester, azelaic acid, hydroquinone, benzoyl peroxide, non-steroidal antiphlogistics, sex hormones, estrogens, androgens, cytostatics, UV protectors, stilbene derivatives, plant extracts such as green tea extract, Centella asiatica extract, willow bark extract, birch extract, tea tree oil, olive leaf extract, Aloe vera extract, marigold extract, passion flower extract, Hamamelis extract, chamomile extract, bearberry leaf extract and licorice root extract, 18β-glycyrhetinic acid (Zn combination), fruit acids such as α-hydroxy acids, β-hydroxy acids, polyhydroxy acids or mixtures thereof.

15. Composition according to one of Claims 11 to 14, **characterized in that** the active component is introduced together with a solubilizing agent.

16. Composition according to one or more of Claims 1 to 15, **characterized in that** the formulation additionally comprises one or more additives useful for a topically applicable composition.

17. Method for the production of an anhydrous multiphase gel composition consisting of an outer lipid matrix and an inner phase gelled by means of a polymer **characterized in that** the lipid phase contains lipids that are compatible with the skin, further **characterized in that** the polymers or polymer blends that can be swollen are swollen by means of swelling agents containing OH groups, further **characterized in that** the swelling agent additionally comprises carbonic acid diesters or mixtures of carbonic acid diesters, wherein
a) the lipid phase is melted with the formation of a liquid lipid phase,
b) polymers or polymer blends capable of swelling are mixed and homogenized with the formation of a polymer phase to be dispersed, wherein the polymer phase comprises the swelling agents containing OH groups and additionally carbonic acid diesters or mixtures of carbonic diesters,
c) the polymer phase is combined with the liquid lipid phase and the phases are homogenized, and
d) the phase mixture is cold stirred until a solid, gel-type mixed structure of the system is formed.

18. Method according to Claim 17, **characterized in that** an active substance is added to the polymer phase, the lipid phase or both phases.

## Revendications

1. Composition en forme d'un système de gel multiphasique anhydre constitué d'une matrice lipidique extérieure et d'une phase intérieure gélifiée par un polymère, **caractérisée en ce que** la phase lipidique contient des lipides compatibles avec la peau, **caractérisée en outre en ce que** les polymères ou mélanges de polymères gonflants sont gonflés au moyen d'agents gonflants contenant des groupes OH, **caractérisée en outre en ce que** l'agent gonflant comprend en outre des diesters d'acide carbonique ou des mélanges de diesters d'acide carbonique pouvant être obtenu par les étapes suivantes :
a) la fusion de la phase lipidique pour former une phase lipidique liquide,
b) le mélange et l'homogénéisation de polymères ou de mélanges de polymères gonflants pour former une phase polymère à disperser qui comprend des agents gonflants contenant des groupes OH et en outre des diesters d'acide carbonique ou des mélanges de diesters d'acide carbonique,
c) la combinaison de la phase polymère avec la phase lipidique liquide et l'homogénéisation des phases, et
d) l'agitation à froid du mélange de phases jusqu"à la formation d'une structure mixte gélifiée solide du système total.

2. Composition selon la revendication 1, **caractérisée en ce que** les lipides sont choisis parmi le pétrolatum, la paraffine, la cire microcristalline, le squalène, l'octanoate de cétylstéaryle, l'oléate d'éthyle, le tricaprylate/caprate de glycéryle (pas 2 composés), le myristate de myristyle, le dicaprate de propylène glycol, les esters cétyliques, le myrisate d'isopropyle, le palmitate d'isopropyle, les mono-, di- et triglycérides, les glycérides éthoxylés, les esters de polyéthylène glycol, les esters de sorbitane, la graisse dure (Novata™), l'adipate de dibutyle, le linoléate d'éthyle, l'isocéteth-3-acétate de propylène glycol (Crodamole), le cocoate d'éthylhexyle, le stéarate d'isocétyle, l'oléate d'oléyle (Cetiol™), le palmitate de cétyle, le palmitate de cétyle (Cutina CP™), l'alcool cétylique, l'alcool oléylique, l'alcool stéarylique, l'éther dicaprylique, l'acide oléique, les cires, la cire de jojoba, la cire d'abeilles, les cholestérols, les polyéthylène glycols, la lanoline, les alcools lanoliniques, les huiles de silicone et leurs mélanges.

3. Composition selon l'une quelconque des revendications précédentes 1 à 2, **caractérisée en ce que** les polymères sont des dérivés de cellulose, des polymères d'acrylate ou leur mélange.

4. Composition selon la revendication 3, **caractérisée en ce que** les dérivés de cellulose sont l'hydroxypropylcellulose, la carboxyméthylcellulose, la méthylcellulose, l'hydroxypropylméthylcellulose, l'hydroxyéthylcellulose ou leur mélange.

5. Composition selon l'une quelconque des revendications 1 à 3, **caractérisée en ce que** les polymères d'acrylate sont des polymères d'acrylate réticulés.

6. Composition selon l'une ou plusieurs des revendications 1 à 5, **caractérisée en ce que** les agents gonflants sont des alcools aliphatiques mono- à trivalents d'une longueur de chaîne de jusqu'à 5 atomes de carbone ou leurs mélanges.

7. Composition selon la revendication 6, **caractérisée en ce que** les alcools aliphatiques monovalents sont choisis parmi l'éthanol, le n-propanol et l'isopropanol ou leur mélange.

8. Composition selon la revendication 6, **caractérisée en ce que** les polyols sont choisis parmi la glycérine, le propylène glycol et le 1,2-pentanediol ou leur mélange.

9. Composition selon l'une ou plusieurs des revendications 1 à 8, **caractérisée en ce que** les diesters d'acide carbonique sont choisis dans le groupe constitué par le carbonate d'éthylène, le carbonate de propylène et les autres homologues de carbonate d'éthylène et leurs mélanges.

10. Composition selon l'une quelconque des revendications 6 à 9, **caractérisée en ce que** l'agent gonflant comprend en outre de l'éther monoéthylique de diéthylène glycol, des glycérides de l'acide caprylcaprique polyoxylés, de l'isosorbide de diméthyle et/ou d'autres solvants pharmaceutiquement compatibles ou leurs mélanges.

11. Composition selon l'une ou plusieurs des revendications 1 à 10, **caractérisée en ce que** la phase lipidique et/ou la phase polymère contiennent en outre des constituants actifs.

12. Composition selon la revendication 11, **caractérisée en ce que** la phase lipidique et la phase polymère contiennent différents constituants actifs.

13. Composition selon la revendication 11 ou la revendication 12, **caractérisée en ce que** les constituants actifs sont choisis parmi les substances de soins de la peau, les substances de coloration de la peau, les protecteurs UV, les substances pharmaceutiquement actives ou leurs mélanges.

14. Composition selon la revendication 11 ou 12, **caractérisée en ce que** les constituants actifs sont choisis parmi le polidocanol, les tannins synthétiques, les antiseptiques, la chlorhexidine, le triclosan, les antibiotiques, l'acide fusidique, l'érythromycine, les tétracyclines, la clindamycine, les antibiotiques peptidiques, les antimycotiques, les dérivés d'imidazole, la terbénafine, le ciclopirox, l'acide salicylique, la pyrithione de zinc, les corticostéroïdes topiques, l'acéponate méthylprednisolone, le clobétasol, le fuorate de mométasone, les macrolides topiques, l'ascrolimus, le tacrolimus, le pimécrolimus, les oligonucléotides pour la thérapie génique, les pARNi, les ribozymes, les antihistaminiques, les immunosuppresseurs, la cyclosporine, l'azathioprine, le mycophénolate mofétil, les anthralines, la cignoline, le dithranol, les analogues de vitamine D3, le calcipotriol, les rétinoïdes topiques, l'urée, l'acide lactique, les esters de l'acide fumarique, l'acide azélaïque, l'hydroquinone, le peroxyde de benzoyle, les antiphlogistiques non stéroïdiens, les hormones sexuelles, les oestrogènes, les androgènes, les cytostatiques, les protecteurs UV, les dérivés de stilbène, les extraits végétaux tels que l'extrait de thé vert, l'extrait de Centella asiatica, l'extrait d'écorce de saule, l'extrait de bouleau, l'huile d'arbre à thé, l'extrait de feuille d'olivier, l'extrait d'aloé vera, l'extrait de souci, l'extrait de fleurs de la passion, l'extrait d'hamamélis, l'extrait de camomille, l'extrait de busserole et l'extrait de racine de réglisse, l'acide 18β-glycyrrhétinique (combinaison Zn), les acides de fruits, tels que les α-hydroxyacides, les β-hydroxyacides, les polyhydroxyacides ou leurs mélanges.

15. Composition selon l'une quelconque des revendications 11 à 14, **caractérisé en ce que** le constituant actif est introduit conjointement avec un agent solubilisant.

16. Composition selon l'une ou plusieurs des revendications 1 à 15, **caractérisée en ce que** la formulation comprend en outre un ou plusieurs additifs utiles pour des compositions applicables topiquement.

17. Procédé de fabrication d'une composition de gel multiphasique anhydre constituée d'une matrice lipidique extérieure et d'une phase intérieure gélifiée par un polymère, **caractérisée en ce que** la phase lipidique contient des lipides compatibles avec la peau, **caractérisée en outre en ce que** les polymères ou mélanges de polymères gonflants sont gonflés au moyen d'agents gonflants contenant des groupes OH, **caractérisée en outre en ce que** l'agent gonflant comprend en outre des diesters d'acide carbonique ou des mélanges de diesters d'acide carbonique, selon lequel :
a) la phase lipidique est fondue pour former une phase lipidique liquide,
b) des polymères ou mélanges de polymères gonflants sont mélangés et homogénéisés pour former une phase polymère à disperser, la phase polymère comprenant les agents gonflants contenant des groupes OH et en outre des diesters d'acide carbonique ou des mélanges de diesters carboniques,
c) la phase polymère est combinée avec la phase lipidique liquide et les phases sont homogénéisées, et
d) le mélange de phases est agité à froid jusqu'à la formation d'une structure mixte gélifiée solide du système.

18. Procédé selon la revendication 17, **caractérisé en ce qu'**un agent actif est ajouté à la phase polymère, à la phase lipidique ou aux deux phases.
